# EUROPEAN PATENT SPECIFICATION

(11) **EP 2 279 762 B1**
(45) Date of publication and mention of the grant of the patent: **20.06.2012**
(21) Application number: 10171066.3
(22) Date of filing: 28.07.2010
(51) Int. Cl.: A61L 15/32, A61L 15/28, A61L 15/44

(54) **Pregelatinized aminoglycosides covalently conjugated to cotton**
Kovalent an Baumwolle konjugierte vorgelatinisierte Aminoglycoside
Aminoglycosides prégélatinisés conjugués de manière covalente au coton

(30) Priority: 30.07.2009 IT MI20091380
(43) Date of publication of application: 02.02.2011
(73) Proprietor: Franzoni Filati S.p.A., 25122 Brescia (IT)
(72) Inventor: Franzoni, Maria Grazia, 25040, CORTE FRANCA (IT); Volpato, Ivo, 06070, CORCIANO (IT); Bizzini, Bernard, 11000, CARCASSONNE (FR)
(74) Representative: Palladino, Saverio Massimo

(56) References cited:
- EP-A2- 0 611 573
- WO-A1-90/15628
- WO-A2-2007/135548

## Description

### Field of the invention

The present invention relates to cotton fibres, yarns or fabrics, to the surface of which pregelatinized aminoglycosides are covalently conjugated, for use in dressing skin lesions.

### Prior art

Skin lesions are alterations to the exterior of the human or animal body, and may be of different types. The first type are wounds, a term which generally means lacerations of the skin caused by external factors such as trauma or surgery. Even burns, while not necessarily incurring skin lacerations, are caused by external events. Conversely, chronic wounds are the result of altered functioning of the skin or internal parts of the body. These include, for example, stasis or decubitus ulcers, ulcers, diabetic ulcers, phlebitis, varicose phlebitis and peripheral thrombosis. In this text, the term "skin lesions" means all the aforementioned types of alterations.

The possible diseases and repair mechanisms related to these alterations vary according to the type of lesion.

Wounds are characterized by immunodefensive, coagulative and reparative processes with normal functionality, relatively short damage repair times and a medium to high risk of microbial contamination. Chronic lesions and burns are characterized generally by poor oxygenation of the injured area, by circulatory and vascular problems, by the presence of tissue necrosis and fibrotic-keratinocyte deposits and, in diabetic ulcers, by reparative dysmetabolism, alterations of peripheral nerve tissue and a very high risk of infections complicated by the possible formation of anaerobiotic pockets. In the treatment of diseases related to skin lesions the aforementioned problems should be taken into account, and hence the treatment means must be able to perform a variety of functions, including providing an adequate protective barrier against infections, allowing proper oxygenation of the involved tissues and supporting the dressing with appropriate and effective therapeutic means (i.e. normocoagulants, or factors that promote revascularization of the treated area, known in the medical field as neoangiogenetic compounds, etc.).

Some known means of wound treatment lead to problems or may even be counterproductive.

For example, the use of gels and ointments is to be seen as a palliative, since the lipid layer typical of these pharmaceutical forms, further to significantly reducing oxygenation of the site, may contribute to the formation of anaerobiotic pockets with a risk of serious infections.

The topical use of antibiotics, for prevention of infections, can reduce the functioning of the subcutaneous reticuloendothelial system and hence impair the immune defence capability.

The use of anti-inflammatory drugs, particularly steroids, also used to relieve pain during dressing application, counters the repair process (reduced neoangiogenesis).

The frequent use of antiseptics, due to their cytotoxic effects, potentiates the necrotizing process and antagonizes macrophage activity.

Also widely used in the treatment of skin lesions are protective means, such as gauzes, compresses, bandages or plasters formed of native cotton and substitutes or the like, possibly previously sterilized, their principal purpose being to mechanically protect the area of the lesion and to form a protective barrier against infections.

These means too are not without potential problems.

In the first instance, they can act as a receptacle for microorganisms which can be easily transferred to the lesion via the exudate and any incompletely coagulated blood. By experimentation, it has even been observed (by exposing them to contaminated sites, after application on agar culture plates) that these means can act as breeding grounds for microorganisms.

This results in the need for their frequent changing, which, because of their ability to function as substrates for cell adhesion, makes them into a means able to promote deposition of necrotic tissue. Again, as a result of their frequent changing, with simultaneous removal of cells undergoing repair, there is an overproduction of exudates causing a reduction in transcutaneous oxygen tension (PtcO₂) and further slowdown of the repair biomechanisms, although pharmacologically promoted.

The final consequences of these processes are an increase in necrotizing phenomena and/or altered immune defences (due to frequent use of antiseptics or antibiotics required for disinfection), increased exudate production, and reduced neoangiogenesis.

In conclusion, it can be stated that the frequency of dressing change, being largely ascribable to the characteristics of the protection means, even if pre-sterilized, is not only at the root of considerable patient discomfort but is also responsible for actually prolonging healing time.

To overcome these problems, "medicated" protections have been proposed i.e. formed of bandages, gauze, compresses and the like, whose surface is functionalized with molecules with pharmacological activity.

Patent application EP 611,573 A2 describes the functionalization of polymers containing numerous free hydroxyl groups, such as cellulose, agarose or polyvinylalcohol, with proteins generally represented by proteolytic enzymes, such as trypsin, chymotrypsin, lysozyme, collagenase, albumin, and hyaluronidase, used to remove pus and necrotic material (chymotrypsin), to reduce the presence of pathogens (lysozyme), and to break down collagen to prevent irregular scarring (collagenase). The polymers, of which cellulose is preferred, may be in powder form but also of fabric suitable for use as bandaging or the like for protecting lesions; functionalization takes place after treatment with N-heterocyclic compounds, in order to separate out the polymer chains and facilitate the reaction between protein amino groups and functional groups on the surface of said chains.

The conjugation of polysaccharides to proteolytic enzymes is also described in patent application CA 1217134 A1. According to the teachings of this document, are functionalized with the enzyme dialdehyde derivatives of dextran or cellulose, in the form of spherules smaller than half a millimeter in order to bring the enzyme into contact with the lesion and fluids present thereon, while preventing it from penetrating the circulation thus avoiding side effects resulting from the antigenic and pyrogenic characteristics of these enzymes. Moreover, this method increases the stability of enzymes in the presence of liquids.

Substances able to provide a substrate for the neoangiogenesis process include proteins and/or their peptide fragments (see for example the article by M.D. Caldwell et al., Progr. in Clin. and Biol. Res., 1991, 365, 205).

A method for functionalizing cellulose-type polymers with proteins is to transform part of the hydroxyl groups of the polymer into aldehyde groups, and then utilize the condensation reaction between amino groups of the protein and carbonyl groups on the polymer, with formation of imine groups (also known as Schiff bases).

Functionalization of polyaldehyde polymers with proteins is however described as (typically) incomplete by S. Margel et al. (J. Polym. Sci Chem., 1984, 22nd Ed, 145) according to which, approximately only a fraction between 0.5% and 10% of free aldehyde groups are involved in the reaction. The residual aldehyde groups may react intramolecularly with amino groups of the bound protein, hence reducing significantly its activity, as described in "Affinity separation with polyaldehyde microsphere beads", S. Margel, J. of Chromatography (1989) 462, pages 177-189.

One possible way of blocking residual aldehyde groups, while at the same time stabilizing said imines, is by a reductive treatment. The reduced polymers however, exhibit a significant reduction in mechanical resistance, greater water solubility and, frequently, a significant reduction in protein activity probably due to the reduction of bonds, such as disulphide bonds, needed for the protein's native activity (L. Peng et al., Appl. Biochem. Biotechnol., 1987, 14, 91).

Another possible way of incorporating bioactive peptides in polymers (in this case, cotton fibres) to confer reparative and antimicrobial properties to biomedical fabrics is described in "Synthesis and formulation of covalently and ionically bound peptide-cellulose conjugates", J. V. Edwards, Bioconjugate Chem., 2000, 17, 469. The method involves the pre-esterification of cellulose with glycine or, alternatively, lysine, histidine and arginine, as a linker between cellulose and an enzyme. Given the lability of the ester bond between the linker and cellulose, the enzyme is however easily released into the site of the lesion.

The use of reduced Schiff bases for the conjugation of bioactive molecules to cellulose fibres is also described in "Immobilization of hyaluronate on cellulose fibres and its use for the isolation of cartilage components", D. L. Kalpaxis et al. Int J Biochem. (1985) 17 (1), 61-66. In this case, hyaluronate is fixed onto a cellulose intermediate derivatized with epsilon amino-caproic acid through a stable bond (reduced Schiff base), obtaining an amide bond between the amino group of the proteoglycan's protein and the carboxyl group of the caproic acid using the carbodiimide technique. The method of reduced Schiff bases is also used in patent application WO 2007/135548 A2, which describes an industrial process for functionalizing cellulose yarns with bioactive molecules using normal plants for dying fabrics.

Finally, the functionalization of polymers (used forthwith for the production of bandages, gauze or the like) with antibiotics is known. These molecules can affect protein synthesis in microorganisms and with their cell membrane function, hence being able to develop their microbicidal function even simply through contact with the microorganism (see the book "The Pharmacological Basis of Therapeutics", Goodman & Gilman, 8th Ed, 1990, 1098-1116).

International patent application WO 90/15628 A1 considers the covalent urethane, ester or amide bond between polymers (preferred is polyethylene glycol, PEG) and antibiotics. These are characterized by a low therapeutic index and/or a very short half-life which precludes their therapeutic use. The method of this document is based on the fact that an antibiotic with a low therapeutic index (eg an antibiotic, such as polymyxin or amphotericin, which is nephrotoxic even at therapeutic doses) may be covalently conjugated to a polymer while allowing the antimicrobial activity to remain unchanged with reduced nephrotoxicity and increased circulating half-life; the aim is therefore to recover those antibiotics, which would otherwise be unusable due to their unfavourable properties, for systemic, oral and/or parenteral therapy.

Finally, another example of conjugating antibiotics to cellulosic fabric materials is described in U.S. Patent 900,803, which teaches the attachment of neomycin to a fabric by using different types of brighteners and/or fixatives. In this case the purpose is to provide anti-odour characteristics to the fabrics. Indeed, it is known that the massive presence of microbial flora is at the root of the malodour during sweating.

Therefore, several systems are known for fixing/conjugating substances having biological/pharmacological activity to cellulosic polymers in the various marketed forms, including for skin lesion treatment. However, in no instance the technique was aimed at a lengthy protection against the risk of infections, possibly with a simultaneous effect on the development of neoangiogenesis and hemostasis.

### Summary of the invention

The object of the present invention is to provide cellulose-based fibres, yarns and fabrics functionalized with antibiotics, which are able to ensure long term protection from infections of skin lesions without the need for frequent dressing changes, while maintaining the necessary levels of oxygen exchange between the environment and the lesion with simultaneous provision of neoangiogenetic factors. A further object of the invention is to provide a process for the production of said fibres, yarns and fabrics.

These aims are achieved with the present invention which, in a first aspect, relates to cotton fibres, yarns and fabrics functionalized simultaneously with at least an aminoglycoside antibiotic (referred to simply as aminoglycosides below) and at least a gelatin.

Functionalization takes place through the formation of covalent bonds between the molecules of antibiotic and gelatin on one side, and cotton on the other side. Aminoglycosides are particularly effective for the purposes of the invention because, besides having an amino functional group required for conjugation with the cotton (as described below), they present a broad spectrum of antimicrobial action and a rapid bactericidal action. Preferred aminoglycosides for the purposes of the invention are gentamicin, kanamycin, tobramycin, amikacin and neomycin. The inventors have observed that by fixing aminoglycosides and gelatin through covalent bonds to cotton (in its various marketed forms corresponding to fibre, yarn, gauze and non-woven fabric), time stable conjugates can be obtained without affecting the range and extent of antibiotic action, and are therefore ideal for obtaining various types of bandaging and other dressings useful for ensuring long periods of protection against risk of infections.

The quantity of aminoglycosides bound to the cotton varies according to the kind of aminoglycoside, its antibiotic activity, and the marketed form of the cotton used.

This quantity is determined, after preliminary tests, in order to have final products containing by weight up to 20 times the MIC value (i.e., the lowest concentration of antibiotic able to kill all pathogenic microorganisms) of the given antibiotic for those bacteria susceptible to the same antibiotic. Preferably, the products of the invention contain 2 to 5 times the MIC value. The reason why, in the products of the invention, antibiotic quantities substantially higher than the MIC value are conjugated to the cotton, is that the yield of the conjugation reaction never reaches 100%, and hence working with higher amounts ensures that there are sufficient quantities of antibiotic on the cotton. Moreover, the aminoglycosides are not equally effective against the entire panel of pathogens susceptible to the antibiotic, and, as a consequence of clinical treatments carried out on sores and wounds, some of these strains may become partially resistant to the antibiotics, this being a previously unforeseeable occurrence. It is therefore better to provide an excess of antibiotics, especially in view of the prolonged contact with those areas to be treated for which the products of the invention are intended.

The inventors have verified by experiment that quantities of aminoglycosides useful for the purposes of the invention are between about 4 and 15 mg of antibiotic (preferably between 6 and 10 mg) per gram of cotton fibre or non-woven fabric, and between about 15 and 45 mg of antibiotic (preferably between 18 and 30 mg) per gram of cotton in the form of gauze or yarn.

Given the analytical difficulties in determining the amount of gelatin that actually binds to cotton during the reaction, the concentration of gelatins in the final product cannot be stated. The inventors have nevertheless verified that useful products for the purposes of the invention are produced by reacting quantities between 0.2 and 5 mg of gelatin, preferably between 0.5 and 1 mg of gelatin, per gram of cotton fibre, and between 0.3 to 6 mg of gelatin, preferably between 0.7 and 1.2 mg of gelatin, per gram of cotton gauze.

The preferred gelatins are those types obtained from bovine or porcine skin, or from porcine bones. Gelatins are commercially available products and are sold for example by the company Sigma-Aldrich.

The products of the invention are capable both of ensuring a lengthy period of protection against the risk of infections, thanks to the presence of the antibiotic, and of providing the optimal peptide substrate for the enzymes present in the lesion exudate, hence giving rise to the production of oligopeptides useful for facilitating the angiogenetic repair process.

The products of the invention can be used for producing protective bandaging for skin lesions in the form of gauze, plasters, or non-woven fabric, representing the second barrier against infections and which are intended for direct contact with the lesion. These products are formed of cotton rendered hydrophilic prior to functionalization.

As is known, these products are generally coupled to a primary barrier, which is not intended for direct contact with the lesion and mainly serves as mechanical protection, in the form of non-fraying bandages or elastic bandages for example, which can be formed from hydrophobic-type cotton derivatives. These primary barriers too can be functionalized with antibiotics to increase the protective effect against any bacteria coming onto the lesion area from outside. To the contrary, in this case the hydrophobic cotton of the primary barrier need not be functionalized additionally with gelatins, since it is not intended for direct contact with the lesion. Covalent conjugation prevents release and/or washout of the antibiotic which protects the lesion, so preventing accumulation of microbial flora, and hence infection, at the site for lengthy periods of time. This enables the need for frequent dressing changes to be reduced, thus facilitating and accelerating the repair process.

In a second aspect, the invention relates to the process of functionalization of cotton with the antibiotic and a gelatin.

The antibiotic and gelatin are attached to the polymer by means of a chemical process involving the following steps:
- bleaching and possible hydrophilization of the cotton in one of the marketed forms of fibre, yarn, cotton, gauze or non-woven fabric;
- oxidation of the previously bleached and possibly hydrophilized cotton, with transformation of hydroxyl groups in the polymer chain of the cotton into carbonyl groups (aldehyde or ketone);
- formation of imines between the amino groups of the antibiotic and of the protein, and the carbonyl groups formed in the previous step;
- reduction of the imine formed in the previous step into the corresponding amine.

The imine formation reaction is carried out simply by using the antibiotic previously dissolved in a gelatin solution.

In the following description, unless otherwise specified, concentrations are to be understood as weight/volume (w/v, i.e. given in grams of solute per 100 ml of solvent) in the case of solid solutes or solutes in gel form like gelatins, antibiotics, sodium metaperiodate, sodium borohydride, lithium aluminium hydride or the like, whereas concentrations are to be understood as volume/volume (v/v) when the solute is H₂O₂, caustic soda (NaOH), Solvipol, acetate and carbonate buffers.

### Detailed description of the invention

All reactions of the process are conducted in typical dyeing vessels, into which the reaction liquids are fed under pressure to the vessel chamber by means of suitable pumps, and extracted at a lower pressure from the centre of the chamber by means of a suction opening. The pressure difference between feed to and extraction from the chamber determines the extent of imbibition by the cotton and hence the extent of reaction, as is well known in the industry. An advantage of the process of the invention is that by operating under the conditions described below, the whole process is conducted in a continuous sequence of steps within the same vessel, with a considerable time and cost saving compared to similar known processes.

The preliminary bleaching step and possible hydrophilization of the cotton is conducted according to well known methods which do not require a detailed description here. This step can be carried out for instance by reacting the cotton (in the pre-selected marketed form) in the presence of hydrogen peroxide, caustic soda, and a detergent, the preferred detergent being Solvipol ECL, supplied by the company Lautex s.r.l. of Grassobbio (BG, Italy). Determining the outcome of this preliminary step, i.e. simple bleaching or bleaching and hydrophilization, is achieved as is well known in the field by controlling process parameters such as sodium hydroxide concentration (this being higher if hydrophilization is required), treatment temperature (for hydrophilization, boiling is required) and contact times with reagents (these being more lengthy in the case of hydrophilization).

The cotton oxidation reaction is carried out preferably with periodic acid or a periodate (preferably sodium metaperiodate) at a temperature generally comprised between 40 and 60 °C for times between about 15 minutes and one hour, with reactor liquid phase feed pressures of between 3 and 5 bar. In this reaction, it is fed into the vessel a solution of periodic acid or periodate such that the active species concentration in the reaction chamber is between about 3 and 10 g/l.

The reaction of imine formation generally takes place at a temperature between about 40 and 60 °C, preferably about 50 °C; under pressure, at pressure values between about 3 and 4 bar, preferably about 3.5 bar; at a pH between 8.0 and 9.0, preferably about 8.5, achieved by adding a carbonate buffer system; and with reaction times between one and five hours.

The antibiotic can be used in free form or in the form of a salt thereof, such as the sulphate in the case of gentamicin.

The gelatin is used preferably in the form of an aqueous solution thereof in a concentration between 0.5 and 3%, preferably about 1%. If using a gelatin solution, the antibiotic or its salt are suspended or dissolved in said solution.

The reaction of imine formation, leading to a covalent conjugation between the cotton and antibiotic plus gelatin is conducted directly in dyeworks vessels, without interruption of the process, after hydrophilization and bleaching of the various items with significant cost savings compared to traditional systems.

Finally, the reaction of imine reduction to the corresponding amine is conducted using methods known in the field, for example at the same pH conditions as the previous step, by adding to the reaction mixture resulting from said reaction a reducing agent such as sodium borohydride (NaBH₄) or lithium aluminium hydride (LiAlH₄) in aqueous solution form, at a concentration of about 1 g/l.

The invention will be further illustrated by means of the following examples.

### Experimental part: Chemistry

### Example 1 (Comparative)

This example relates to the conjugation of gentamicin to cotton fibre rendered hydrophilic.

A forced circulation dyeing vessel is used, having a total vessel chamber volume of about 100 I and an operational volume of 60 I (the operational volume is a technological value provided by the manufacturer of the vessel, and is the maximum volume of liquid that can be safely fed into it). 10 kg of cotton fibre are loaded into the vessel and water is allowed to circulate continuously for ten minutes for correct imbibition. The following procedure is then carried out:
- hydrophilization and bleaching of the fibre
   the vessel is brought to its rated volume, i.e. it is filled up to the operational volume of 60 I with deionized water, and the following are added:
- 10 g/l of hydrogen peroxide at 35% concentration;
- 4 g/l of caustic soda at 30% concentration;
- 1 g/l of the detergent Solvipol ECL.
   The bath temperature is brought to 102 °C and the reaction is allowed to proceed, still in forced circulation, for 30 minutes. A series of continuous washes is then performed until the washing waters are neutralized;
- oxidation of the bleached and hydrophilized fibre
   the reaction chamber is filled and the pH is adjusted to 5.6 by adding acetate buffer. The temperature is brought to 50 °C and pressure to 3.5 bar. Sodium metaperiodate is added in a quantity such that its concentration in the reaction mixture is equal to 5.32 g/l and is allowed to react for 30 minutes (controlled oxidation). Two continuous washes lasting ten minutes are then carried out;
- functionalization with the antibiotic
   the reaction chamber is again filled, the temperature is again brought to 50 °C and the pressure to 3.5 bar; the pH is then adjusted to 8.5 by adding carbonate buffer. Gentamicin sulphate is added, pre-solubilized in water at a quantity such that its concentration in the reaction mixture is equal to 0.8 g/l, and the reaction is allowed to proceed for three hours. In this reaction the imine (Schiff base) forms between the aldehyde groups of the oxidized cotton and an amino group of gentamicin. Two continuous washes are carried out for ten minutes each;
- reduction of the obtained imine groups to amines
   the vessel is filled again and the pH returned to 8.5 by adding a carbonate buffer; sodium borohydride is added in a quantity such that its concentration in the reaction mixture is equal to 1.0 g/l, and the reaction is allowed to proceed at room temperature for 15 minutes. In this reaction the imine is reduced to the corresponding alkylamine. Three continuous washes are carried out for ten minutes each and the fibre is unloaded and dried in a hot air tunnel at a temperature of about 80-90 °C.

The thus obtained hydrophilic fibre, bleached and conjugated to gentamicin, is sample 1. This fibre is used to create fluff for patches and protective non-woven fabrics.

### Example 2

This example relates to the conjugation of pregelatinized gentamicin to cotton fibre rendered hydrophilic.

The procedure of hydrophilization, bleaching and oxidation of 10 kg of cotton fibres as described in Example 1 is repeated, except that sodium metaperiodate is used in the oxidation step in a quantity such that its concentration in the reaction mixture is equal to 3.8 g/l.

In a separate vessel, gentamicin sulphate pregelatinization is carried out. For this purpose, 100 g of gentamicin sulphate are dissolved in 1 litre of a 1% concentration aqueous solution of type B gelatin, from bovine skin (225 Bloom, sold by Sigma Aldrich Chemical Company), maintaining it under agitation at a temperature of 50 °C. The reaction mixture is maintained at this temperature until its use in the conjugation reaction on cotton.

The previously prepared hydrophilized, bleached and oxidized fibre is reacted with the pregelatinized antibiotic. The reaction chamber containing the fibre is heated to a temperature of 50 °C and the pressure is set at 3.5 bar, then the pH is adjusted to 8.5 by adding carbonate buffer. The pregelatinized gentamicin is added in a quantity such that the concentration of gentamicin sulphate is equal to 1.4 g per litre of reaction mixture and the mixture is allowed to react for three hours with formation of imines between the aldehyde groups of oxidized cotton and an amino group of gentamicin and part of the gelatin amino groups. Two continuous washes of ten minutes each are performed.

Finally the reduction with sodium borohydride is repeated as described in Example 1. That part of the gelatin used which is not conjugated remains trapped, even after washing, between the cotton fibre strands. Three continuous washes are performed for ten minutes each and the fibre is unloaded then dried in an air tunnel at 40 °C.

The thus obtained hydrophilic fibre, bleached and conjugated to gelatinized gentamicin, is the sample 2; this fibre too is used to produce fluff for plasters and protective non-woven fabrics.

### Example 3 (Comparative)

This example relates to the conjugation of gentamicin to a hydrophobic cotton yarn.

10 kg of yarn in bobbins are loaded into a 100 I forced circulation yarn dyeing vessel and water is circulated continuously for 10 minutes for correct imbibition of the yarn. 15% sodium hypochlorite (20 g/I) and caustic soda 36 BE (1.5 g/I) are added to the yarn, and the reaction is allowed to proceed for one hour at 60 °C. The yarn is repeatedly washed and 30 vol hydrogen peroxide (6 g/I) and caustic soda 36 BE (2 g/I) are then added allowing the reaction to proceed for a further hour, followed by repeated washing until the washing water is neutralized. Oxidation of the cotton then ensues as described in the process of Example 1, with the only differences that sodium metaperiodate is used in a quantity such that its concentration in the reaction mixture is equal to 5 g/l, and that the reaction is allowed to continue for 15 minutes. Under these conditions the oxidation is controlled, with the aim of maintaining the market properties necessary for weaving the yarn. Two continuous washes are performed for ten minutes each. The functionalization with gentamicin procedure of Example 1 is then repeated, with the only difference being that the gentamicin sulphate is used in a quantity such that its concentration in the reaction mixture is 1.6 g/l. Two continuous washes are performed for ten minutes each.

Finally, the reduction procedure of Example 1 is repeated, and the bobbins of yarn are unloaded and dried in a hot air tunnel at 80 to 90 °C.

The yarn thus obtained, bleached and conjugated with gentamicin, is the sample 3. This yarn is woven to create hemmed bandages and elastic bandages for the external protection of skin wounds and lesions.

### Example 4

This example relates to the conjugation of pregelatinized gentamicin to cotton gauze rendered hydrophilic. 20 kg of cotton gauze in rolls 150 cm in width are loaded into a 200 I forced circulation fabric dyeing vessel (so-called beam dyeing autoclave). Water is allowed to circulate continuously for ten minutes for correct imbibition of the gauze.

The hydrophilization, bleaching and oxidation procedure of Example 1 is repeated with the only difference being that in the oxidation step sodium metaperiodate is used in a quantity such that its concentration in the reaction mixture is equal to 6.2 g/l.

Separately, pregelatinization of gentamicin sulphate is carried out, repeating the procedure described in Example 2 for this purpose, the only difference being that in this case 500 g of gentamicin sulphate are used. The reaction mixture is maintained at this temperature until its use in the conjugation reaction on cotton. The hydrophilized and bleached gauze obtained as described above is reacted with the pregelatinized antibiotic, repeating the procedure of Example 2 with the only difference being that the pregelatinized gentamicin is added in such a quantity that the concentration of gentamicin sulphate is equal to 2.6 g per litre of reaction mixture. Two continuous washes are performed for ten minutes each. Finally, the procedure of imine reduction of Example 2 is repeated. Again, part of the gelatin used but not conjugated remains trapped, even after washing, in the cotton gauze. Three continuous washes are performed for ten minutes each and the gauze roll is unloaded and dried by unwinding the roll and simultaneously passing it through a hot air tunnel.

The thus obtained hydrophilic gauze, bleached and conjugated to gelatinized gentamicin is the sample 4. This gauze, after cutting to the appropriate size, is used as a second protective layer in direct contact with wounds or lesions.

### Example 5

This example relates to the conjugation of pregelatinized kanamycin to a cotton fibre rendered hydrophilic.

The procedure of Example 2 is repeated, with the only differences being that kanamycin sulphate is used instead of gentamicin sulphate, and that in the functionalization of the fibre, a quantity of pregelatinized kanamycin is used such that the concentration of kanamycin sulphate is equal to 1 gram per litre of reaction mixture. Again, part of the gelatin used but not conjugated remains trapped, even after washing, between the strands of cotton fibres. Three continuous washes are performed for ten minutes each and the fibre is unloaded and dried in an air tunnel at 40 °C.

The thus obtained hydrophilic fibre, bleached and conjugated to gelatinized kanamycin, is the sample 5. This fibre is used to produce fluff for plasters and protective non-woven fabrics intended for direct contact with the lesion.

### Example 6 (Comparative)

This example relates to the conjugation of kanamycin to a hydrophobic cotton yarn.

The procedure of Example 3 is repeated, with the only differences being that kanamycin is used instead of gentamicin, and that in the functionalization with antibiotic, kanamycin sulphate is used in quantities such that its concentration in the reaction mixture is equal to 1.2 g/l.

The thus obtained yarn, bleached and conjugated to kanamycin, is the sample 6, and is employed for the same uses as the yarn in Example 3.

### Example 7

This example relates to the conjugation of pregelatinized kanamycin to cotton gauze rendered hydrophilic.

The procedure in Example 4 is repeated, with the only differences being that kanamycin is used instead of gentamicin, and that in the functionalization of the gauze a quantity of pregelatinized kanamycin sulphate is used such that the concentration of kanamycin sulphate in the reaction mixture is equal to 1.8 g/l. Again, part of the gelatin used but not conjugated remains trapped, even after washing, in the cotton gauze.

The thus obtained gauze, bleached and conjugated to gelatinized kanamycin, is the sample 7. This gauze, after cutting to the appropriate size, is used as the second protective layer in direct contact with wounds or lesions.

### Example 8

This example relates to the conjugation of pregelatinized amikacin to cotton fibre rendered hydrophilic.

The procedure in Example 2 is repeated, with the only differences being that amikacin sulphate is used instead of gentamicin sulphate, and that in the functionalization of the fibre a quantity of pregelatinized amikacin is used such that the concentration of amikacin sulphate is equal to 1.5 g per litre of reaction mixture. Again, part of the gelatin used but not conjugated remained trapped, even after washing, between the cotton fibre strands.

The thus obtained hydrophilic fibre, bleached and conjugated to gelatinized amikacin, is the sample 8. This fibre is used to produce fluff for plasters and protective non-woven fabric intended for direct contact with the lesion.

### Example 9 (Comparative)

This example relates to the conjugation of amikacin to hydrophobic cotton yarn.

The procedure in Example 3 is repeated, with the only differences being that amikacin is used instead of gentamicin, and that in the functionalization with antibiotic, amikacin sulphate is used in a quantity such that its concentration in the reaction mixture is 2.0 g/l.

The thus obtained yarn, bleached and conjugated to amikacin, is the sample 9, and is employed for the same uses as the yarn in Example 3.

### Example 10

This example relates to the conjugation of pregelatinized amikacin to cotton gauze rendered hydrophilic.

The procedure in Example 4 is repeated, with the only differences being that amikacin is used instead of gentamicin, and that in the functionalization of the gauze pregelatinized amikacin sulphate is used in a quantity such that the concentration of amikacin sulphate in the reaction mixture is equal to 3.0 g/l. Again, part of the gelatin used but not conjugated remains trapped, even after washing, in the cotton gauze.

The thus obtained gauze, bleached and conjugated to gelatinized amikacin, is the sample 10, and is employed for the same uses as the gauze in Example 4.

### Experimental part: Pharmacology

### Example 11

This example relates to the inhibition of Gram + and Gram - bacteria growth on nutrient agar plates by the samples of the invention.

A series of 9 cm diameter Petri dishes containing sterile nutrient agar is prepared in which 20 µl of a fresh culture of S. aureus or E. coli are introduced. After cooling, a 3 cm diameter disk of samples 1-10, prepared as described in the previous examples, is placed on the agar surface.

The plates are incubated at 37 °C for 18 hours after which the number of CFUs (colony forming units) of pathogenic microorganisms is examined on the surface of the culture medium next to the cotton disk as well as any zone of inhibition (ZI, in millimeters) which, if present, indicates the release of antibiotic into the culture medium.

In parallel, some plates are prepared onto which a disk of unconjugated cotton (control) is placed.

The results are presented in Table 1.

**Table 1**

| Sample | | Results | | | |
|---|---|---|---|---|---|
| No. | Structure | S. aureus | | E. coli | |
| | | CFU (no.) | ZI (mm) | CFU (no.) | ZI (mm) |
| 1 | Hydrophilic fibre with gentamicin | 0 | 2 | 0 | 1 |
| 2 | Hydrophilic fibre with gelatinized gentamicin | 0 | 0 | 0 | 0 |
| 3 | Hydrophobic yarn with gentamicin | 0 | 1 | 0 | 0 |
| 4 | Hydrophilic gauze with gelatinized gentamicin | 0 | 0 | 0 | 0 |
| 5 | Hydrophilic fibre with gelatinized kanamycin | 0 | 0 | 0 | 1 |
| 6 | Hydrophobic yarn with kanamycin | 0 | 2 | 0 | 2 |
| 7 | Hydrophilic gauze with gelatinized kanamycin | 0 | 0 | 0 | 0 |
| 8 | Hydrophilic fibre with gelatinized amikacin | 0 | 0 | 0 | 0 |
| 9 | Hydrophobic yarn with amikacin | 0 | 1.5 | 0 | 1 |
| 10 | Hydrophilic gauze with gelatinized amikacin | 0 | 0 | 0 | 0 |
| / | Untreated hydrophilic cotton gauze (control) | > 1000 | / | > 1000 | / |
| / | Untreated hydrophobic cotton bandage (control) | > 1000 | / | > 1000 | / |

The results show that, for all the samples of the invention conjugated to aminoglycoside antibiotics, whether hydrophilic cotton-based or hydrophobic cotton-based, and in the presence or absence of gelatin, there is no microbial growth (Gram + and Gram -) at their surfaces. This demonstrates the complete microbicidal activity of the antibiotics, conjugated to cotton, in the two different forms, and the fact that gelatinization does not negatively affect the microbicide properties of the antibiotics.

The reduced entity, and in most cases the absence of an inhibition zone of microbial growth around the surface of the disks of conjugated cotton, demonstrates the non-release of the antibiotics into the growth medium, this being indicative of a stable conjugation bond.

### Example 12

This example relates to inhibition of Gram + and Gram - bacteria growth on nutrient broth.

Wads of cotton conjugated to antibiotics, weighing about one gram and corresponding to samples 1-10, are placed into microbiology tubes containing 20 ml of sterile nutrient broth, above the level of the broth.

On top of these 20 µl of a fresh culture of S. aureus or E. coli are deposited, and maintained in contact for one hour. The wads are then immersed in the culture broth and the tubes are incubated at 37 °C for 18 hours. At the end of incubation, the wads are removed from the tubes and the intensity of culture development is determined by a spectrophotometric opalescence reading (O.D.) at 640 nm.

In parallel, growth tests without cotton wads (growth controls) and tests using untreated cotton wads (reference controls) are carried out.

The results are reported in table 2

**Table 2**

| Sample | | Results | |
|---|---|---|---|
| | | (O.D. at 640 nm) | |
| No. | Structure | S. aureus | E. coli |
| 1 | Hydrophilic fibre with gentamicin | 0.020 | 0.034 |
| 2 | Hydrophilic fibre with gelatinized gentamicin | 0.036 | 0.024 |
| 3 | Hydrophobic yarn with gentamicin | 0.018 | 0.022 |
| 4 | Hydrophobic gauze with gelatinized gentamicin | 0.030 | 0.030 |
| 5 | Hydrophilic fibre with gelatinized kanamycin | 0.016 | 0.024 |
| 6 | Hydrophobic yarn with kanamycin | 0.026 | 0.036 |
| 7 | Hydrophilic gauze with gelatinized kanamycin | 0.014 | 0.026 |
| 8 | Hydrophilic fibre with gelatinized amikacin | 0.020 | 0.020 |
| 9 | Hydrophobic yarn with amikacin | 0.030 | 0.015 |
| 10 | Hydrophilic gauze with gelatinized amikacin | 0.026 | 0.018 |
| / | Growth control | 0.540 | 0.500 |
| / | Untreated hydrophilic cotton gauze (control) | 0.960 | 0.880 |
| / | Untreated hydrophobic cotton bandage (control) | 0.750 | 0.800 |

The results demonstrate that a contact time of one hour between the various aminoglycoside antibiotics conjugated to the various hydrophilic and hydrophobic forms of cotton, either with or without pregelatinization thereof, with Gram + and Gram - pathogenic microorganisms, is sufficient for complete inhibition of their growth. In this case too, then, evidence is obtained that gelatinization of the samples does not negatively affect the microbicide properties of the antibiotic.

In contrast, when the micro-organic inoculum is in contact with normal control cotton in the form of bandaging, a significant increase is noted in microbial growth compared to the growth control. This is evidence that normal cotton not only acts as an accumulator of microbial flora but also as a catalyst for their growth.

### Example 13

This example relates to the inhibition of Gram + and Gram - bacteria growth on nutrient broth after prolonged contact with blood and artificial sweat. Approximately 2 g samples of cotton gauze and bandages conjugated to antibiotics, corresponding to samples 1-10, are immersed for six days in whole citrated pig's blood, which is shaken occasionally. The samples are then recovered, washed with water until blood residues are removed, immersed in artificial sweat (R. Selleri et al., The Chemistry and Technology of Cosmetic Products, 1977, 11) and maintained therein, again shaking occasionally, for a further four days.

At the end of the treatment, the samples are repeatedly washed with water, dried in a hot air current then tested for antimicrobial activity against S. aureus and E. coli according to the method described in the preceding example.

The results are reported in table 3.

**Table 3**

| Sample | | Results | |
|---|---|---|---|
| | | (O.D. at 640 nm) | |
| No. | Structure | S. aureus | E. coli |
| 1 | Hydrophilic fibre with gentamicin | 0.040 | 0.036 |
| 2 | Hydrophilic fibre with gelatinized gentamicin | 0.024 | 0.038 |
| 3 | Hydrophobic yarn with gentamicin | 0.054 | 0.046 |
| 4 | Hydrophobic gauze with gelatinized gentamicin | 0.060 | 0.034 |
| 5 | Hydrophilic fibre with gelatinized kanamycin | 0.052 | 0.048 |
| 6 | Hydrophobic yarn with kanamycin | 0.072 | 0.080 |
| 7 | Hydrophilic gauze with gelatinized kanamycin | 0.040 | 0.050 |
| 8 | Hydrophilic fibre with gelatinized amikacin | 0.034 | 0.060 |
| 9 | Hydrophobic yarn with amikacin | 0.065 | 0.070 |
| 10 | Hydrophilic gauze with gelatinized amikacin | 0.046 | 0.054 |
| / | Growth control | 0.480 | 0.520 |

The results show indirectly that the various aminoglycoside antibiotics conjugated to hydrophilic and hydrophobic cotton, either as such or in the presence of gelatin, are not released after a significant contact period with blood and sweat, correlatable to the times used with dressings, thus ensuring long term conservation of microbicidal activity under conditions of actual use. In this case too, then, evidence is obtained that gelatinization of the samples does not negatively affect the microbicide properties of the antibiotic.

### Example 14

This example relates to the prevention of S. epidermidis infections in injured rat skin.

Female Wistar rats weighing about 200 g, five per group, are suitably depilated in the dorsal region and then, under light anaesthesia, are subjected to prolonged exposure to UV rays until evident lesions are induced on the skin.

Disks of cotton gauze or bandage conjugated to various antibiotics, produced according to examples 1-10, are applied to the injured area using adhesive, and are maintained in contact in situ for ten days. Each day, 5 µl/site of a fresh S. epidermidis culture are applied at three different points to the surface of the disks. At the end of the treatment, the disks are removed and the infective condition of the lesions is examined by drawing off the exudate with a swab and inoculating culture broth therewith. At the same time a subjective examination of the condition of the lesion itself is undertaken.

A parallel treatment is carried out on a group of animals by applying disks of unconjugated cotton, gauze or bandage (controls).

The results are reported in table 4. The data for microbial growth are given as the number of animals presenting with a S. epidermidis infection, verified by taking a swab from the lesion and examining the growth in culture broth and by Gram staining, over the total number of animals tested (n/n). The results, reported in the column "Lesion condition", are given in the form of subjective indicators of lesion severity, in accordance with the following scale: (+++) necrotic, (++) very severe, (+) severe, (-) improved, (--) much improved, (---) considerably improved.

**Table 4**

| Sample | | Results | |
|---|---|---|---|
| No. | Structure | Microbial growth | Lesion condition |
| 1 | Hydrophilic fibre with gentamicin | 0/5 | (--) |
| 2 | Hydrophilic fibre with gelatinized gentamicin | 0/5 | (---) |
| 3 | Hydrophobic yarn with gentamicin | 0/5 | (-) |
| 4 | Hydrophilic gauze with gelatinized gentamicin | 0/5 | (---) |
| 5 | Hydrophilic fibre with gelatinized kanamycin | 0/5 | (---) |
| 6 | Hydrophobic yarn with kanamycin | 0/5 | (-) |
| 7 | Hydrophilic gauze with gelatinized kanamycin | 0/5 | (---) |
| 8 | Hydrophilic fibre with gelatinized amikacin | 0/5 | (---) |
| 9 | Hydrophobic yarn with amikacin | 0/5 | (-) |
| 10 | Hydrophilic gauze with gelatinized amikacin | 0/5 | (---) |
| / | Untreated hydrophilic cotton gauze (control) | 5/5 | (+++) |
| / | Untreated hydrophobic cotton bandage (control) | 5/5 | (++) |

The results show that, in contrast to application of normal control cotton where lesion infections are present in all the treated animals, conjugates between the various aminoglycosides and the hydrophilic and hydrophobic cotton, either as such or in gelatinized form, are able to provide total protection from infections.

A subjective examination of the lesion condition, inspected on removal of the protective disk, shows that conjugated between cotton and gelatinized antibiotics allow a particularly evident improvement in erythema severity such as to be almost healed; in contrast, conjugated between cotton and the sole antibiotics, while avoiding microbial growth, give rise to recovery of the skin damage of much lesser extent.

### Example 15

This example relates to the analysis of haemostatic properties in rats.

Female Wistar rats weighing about 300 grams are divided into groups of eight units each.

In each animal, after ether anaesthesia, a 2 cm long deep vertical incision is made with a razor blade in the caudal vein, such that each animal has as far as possible the same wound and consequent blood flow. Subsequently, strips of the various cotton gauze samples under study are applied and maintained in close contact until bleeding has stopped (which on average takes two to four minutes). Following their removal, the bloodstain size and condition of the clot on the various gauze samples are examined, the latter subjectively (blood more or less dried, firmness of the platelet plug).

Over the next six hours the animals are checked for possible resumption of bleeding and firmness of the clot in formation.

The results are presented in Table 5 which reports the results relating to the bloodstain on the sample (in cm²), bleeding resumption data (as a % of the number of animals), and firmness of the clot, assessed using the following subjective scale: + + + = very firm and dry clot; + + = firm and moist clot, + = weak clot.

**Table 5**

| Sample | | Results | | |
|---|---|---|---|---|
| No. | Structure | Bloodstain (cm²) | Bleeding resumption (% animals) | Clot firmness |
| 1 | Hydrophilic fibre with gentamicin | 4.2 | 0.0 | ++ |
| 2 | Hydrophilic fibre with gelatinized gentamicin | 1.9 | 0.0 | +++ |
| 3 | Hydrophobic yarn with gentamicin | 4.5 | 12.5 | + |
| 4 | Hydrophilic gauze with gelatinized gentamicin | 2.0 | 0.0 | +++ |
| 5 | Hydrophilic fibre with gelatinized kanamycin | 2.3 | 0.0 | +++ |
| 6 | Hydrophobic yarn with kanamycin | 4.8 | 25.0 | + |
| 7 | Hydrophilic gauze with gelatinized kanamycin | 1.8 | 0.0 | +++ |
| 8 | Hydrophilic fibre with gelatinized amikacin | 2.2 | 0.0 | +++ |
| 9 | Hydrophobic yarn with amikacin | 5.4 | 12.5 | + |
| 10 | Hydrophilic gauze with gelatinized amikacin | 2.6 | 0.0 | +++ |
| / | Untreated hydrophilic cotton gauze (control) | 4.8 | 12.5 | + |
| / | Untreated hydrophobic cotton bandage (control) | 6.0 | 25.0 | + |

The results for the bloodstain derived from the wound demonstrate a significant acceleration of hemostasis and an equally significant reduction in hemorrhagic phenomena for samples containing the gelatinized aminoglycoside antibiotic compared to those containing the antibiotic alone.

The hemostatic effect of the conjugates of the invention is confirmed by a total absence of bleeding resumption and an evident firmer hemostatic plug found in animals treated with this type of protection.

### Experimental part: Clinical

### Example 16

This example relates to preliminary studies on usage for surgical wounds. Cotton-based protective bandaging (gauze, bandages and plasters) covalently conjugated with aminoglycosides and pregelatinized aminoglycosides were compared with gauze, bandages and plasters in normal use in clinical-surgical practice on a significant number of patients undergoing small to medium-sized surgical procedures.

The wounds were protected by applying, in direct contact thereto, hydrophilic cotton-based gauze conjugated to the gelatinized antibiotic (second antimicrobial protective barrier) which in turn was covered with hydrophobic cotton-based bandages conjugated to the same non-gelatinized antibiotic (first antimicrobial protective barrier).

Whereas the normal protective bandaging (controls) were replaced every twenty-four or forty-eight hours in accordance with routine practice, with simultaneous normal disinfection of the wound, the articles of the invention were left to protect the injured site throughout the period required for the wound to heal, on average comprised between eight and twelve days.

By using the articles produced according to the invention, no cases of infection were present in the application site. Furthermore, compared to the values obtained with control bandaging, the articles produced according to the invention were faster and better at haemostasis and tissue repair (healing).

### Example 17

This example relates to preliminary studies of use with chronic skin lesions. Protective bandaging (gauze, bandages) produced in accordance with the invention were compared with standard gauze and bandages, for protection of burns or bedsores on a significant number of patients.

The lesion was protected by applying in direct contact thereto gauze of hydrophilic cotton conjugated to the gelatinized antibiotic (second antimicrobial protective barrier) which in turn was covered with hydrophobic cotton-based bandages conjugated to the same non-gelatinized antibiotic (first antimicrobial protective barrier).

Whereas the normal protective articles (controls) were replaced on average every forty-eight hours in accordance with routine practice, with simultaneous normal disinfection of the wound, the articles of the invention were replaced on average every ten/twelve days. The treatment was continued, depending on the nature of the lesion, for up to six months.

By using the articles prepared according to the invention, no cases of infection were present at the protected lesion; conversely, where normal articles were used, a thorough disinfection had to be performed in a large number of subjects. Significantly extending the replacement intervals of protective articles has resulted in a significant improvement in the general condition of the lesions compared with controls, these being subject to frequent replacement. With the articles produced according to the present invention, a significant regression of the lesion was actually noted with an absence of necrotic sites and a tendency for exudation to normalize.

## Claims

1. Product based on hydrophilic cotton, functionalized with at least one aminoglycoside antibiotic and at least a gelatin, **characterized in that** the antibiotic and the gelatin are bound to the cotton by means of a covalent bond, the antibiotic is present in a quantity comprised between about 4 and 45 mg per gram of cotton, and said product is obtained by reacting the cotton with quantities of at least a gelatin comprised between 0.2 and 6 mg per gram of cotton.

2. Product according to claim 1, wherein the antibiotic is one or more chosen from gentamicin, kanamycin, tobramycin, amikacin and neomycin.

3. Product according to claim 1, wherein the cotton is present in the form of fibre, yarn, gauze or non-woven fabric.

4. Product according to claim 3, wherein the quantity of antibiotic is comprised between 4 and 15 mg per gram of cotton when this latter is in the form of fibre.

5. Product according to claim 4, wherein said quantity of antibiotic is comprised between 6 and 10 mg per gram of cotton.

6. Product according to claim 3, wherein the quantity of antibiotic is comprised between 14 and 45 mg per gram of cotton when this latter is in the form of gauze.

7. Product according to claim 6, wherein said quantity of antibiotic is comprised between 18 and 30 mg per gram of cotton.

8. Product according to claim 1, wherein said gelatins are obtained from bovine or porcine skin, or porcine bone parts.

9. Product according to claim 1, wherein the quantity of gelatins is comprised between 0.2 and 5 mg per gram of cotton when this latter is in the form of fibre.

10. Product according to claim 9, wherein said quantity of gelatins is comprised between 0.5 and 1 mg per gram of cotton.

11. Product according to claim 1, wherein the quantity of gelatins is comprised between 0.3 and 6 mg per gram of cotton when this latter is in the form of gauze.

12. Product according to claim 11, wherein said quantity of gelatins is comprised between 0.7 and 1.2 mg per gram of cotton.

13. Process for producing a product according to any one of the preceding claims, based on hydrophobic cotton, comprising the following steps:
- bleaching of the cotton in one of the marketed forms: fibre, yarn, gauze or non-woven fabric;
- oxidation of the previously bleached cotton, with transformation of the hydroxyl groups present in the polymer chain of the cotton into carbonyl groups (aldehyde or ketone);
- formation of imines between the amino groups of the antibiotic and of the gelatin and the carbonyl groups formed in the previous step;
- reduction to amines of the imines formed in the previous step.

14. Process for the production of a product according to any one of claims 1-12, based on hydrophilic cotton, comprising the following steps:
- bleaching and hydrophilization of the cotton in one of the marketed forms: fibre, yarn, gauze or non-woven fabric;
- oxidation of the cotton previously hydrophilized and bleached, with transformation of the hydroxyl groups present in the polymer chain of the cotton into carbonyl groups (aldehyde or ketone);
- formation of imines between the carbonyl groups formed in the previous step and the amino groups of the antibiotic and of the gelatin;
- reduction of the imines formed in the previous step to amines.

15. Process according to any one of claims 13 or 14, wherein the bleaching of the cotton is carried out by reacting the cotton with hydrogen peroxide, caustic soda and a detergent.

16. Process according to any one of claims 13 or 14, wherein the oxidation of the cotton is carried out with periodic acid or a periodate with a concentration comprised between 3 and 10 g/l, at a temperature comprised between 40 and 60 °C, for periods comprised between about 15 minutes and 1 hour, and with reactor liquid phase feed pressures of between 3 and 5 bar.

17. Process according to any one of claims 13 or 14, wherein formation of the imines takes place at a temperature comprised between about 40 and 60 °C, with pressure values comprised between about 3 and 4 bar, at a pH comprised between 8.0 and 9.0, and with reaction times comprised between 1 and 5 hours, and wherein the antibiotic is used in free form or as a salt thereof.

18. Process according to claim 14, wherein the formation of the imines is carried out by reacting the carbonyl groups of cotton and the amino groups of the antibiotic and of the gelatin proteins at a temperature comprised between about 40 and 60 °C, with pressure values comprised between about 3 and 4 bar, at a pH comprised between 8.0 and 9.0, and with reaction times comprised between 1 and 5 hours, and wherein the antibiotic is used in a free form or as a salt thereof.

19. Process according to claim 18, wherein the formation of the imines is carried out by using the antibiotic or a salt thereof previously dissolved or suspended in an aqueous solution of one or more gelatins, wherein the total concentration of said gelatins is comprised between 0.5 and 3% w/v.

20. Process according to any one of claims 13 or 14, wherein the reduction of the imines to amines is carried out by adding to the reaction mixture resulting from the previous step a reducing agent such as sodium borohydride (NaBH₄) or lithium aluminium hydride (LiAlH₄) in the form of aqueous solutions having a concentration of 1 g/l.

## Patentansprüche

1. Produkt basierend auf hydrophiler Baumwolle, funktionalisiert mit mindestens einem Aminoglycosid-Antibiotikum und mindestens einer Gelatine, **dadurch gekennzeichnet, dass** das Antibiotikum und die Gelatine über eine kovalente Bindung an die Baumwolle gebunden sind, wobei das Antibiotikum in einer Menge, umfassend zwischen 4 und 45 mg pro Gramm Baumwolle, vorliegt, und genanntes Produkt durch die Reaktion der Baumwolle mit Mengen an Gelatine, umfassend zwischen 0,2 und 6 mg pro Gramm Baumwolle, erhalten wird.

2. Produkt gemäß Anspruch 1, wobei es sich bei dem Antibiotikum um ein Antibiotikum oder mehrere Antibiotika, ausgewählt aus Gentamicin, Kanamycin, Tobramycin, Amikacin und Neomycin handelt.

3. Produkt gemäß Anspruch 1, wobei die Baumwolle in der Form von Fasern, Garn, Gaze oder nicht gewebtem Gewebe vorliegt.

4. Produkt gemäß Anspruch 3, wobei die Menge an Antibiotikum zwischen 4 und 15 mg pro Gramm Baumwolle umfasst, wenn letztere in der Form einer Faser vorliegt.

5. Produkt gemäß Anspruch 4, wobei genannte Menge an Antibiotikum zwischen 6 und 10 mg pro Gramm Baumwolle umfasst.

6. Produkt gemäß Anspruch 3, wobei die Menge an Antibiotikum zwischen 14 und 45 mg pro Gramm Baumwolle umfasst, wenn letztere in der Form von Gaze vorliegt.

7. Produkt gemäß Anspruch 6, wobei genannte Menge an Antibiotikum zwischen 18 und 30 mg pro Gramm Baumwolle umfasst.

8. Produkt gemäß Anspruch 1, wobei genannte Menge an Gelatinen aus Rinder- oder Schweinehaut oder Teilen von Schweineknochen gewonnen wird.

9. Produkt gemäß Anspruch 1, wobei genannte Menge an Gelatinen zwischen 0,2 und 0,5 mg pro Gramm Baumwolle umfasst, wenn letztere in der Form von Fasern vorliegt.

10. Produkt gemäß Anspruch 9, wobei genannte Menge an Gelatinen zwischen 0,5 und 1 mg pro Gramm Baumwolle umfasst.

11. Produkt gemäß Anspruch 1, wobei die Menge an Gelatinen zwischen 0,3 und 6 mg pro Gramm Baumwolle umfasst, wenn letztere in der Form von Gaze vorliegt.

12. Produkt gemäß Anspruch 11, wobei genannte Menge an Gelatinen zwischen 0,7 und 1,2 mg pro Gramm Baumwolle umfasst.

13. Verfahren zur Herstellung eines Produkts gemäß einem der vorherigen Ansprüche basierend auf hydrophober Baumwolle, umfassend die folgenden Schritte:
- Bleichen der Baumwolle in einer der vermarkteten Formen: Faser, Garn, Gaze oder nicht gewebtes Gewebe;
- Oxidation der zuvor gebleichten Baumwolle mit Umwandlung der in der Polymerkette der Baumwolle vorhandenen Hydroxylgruppen in Carbonylgruppen (Aldehyd oder Keton);
- Bildung von Iminen zwischen den Aminogruppen des Antibiotikums und der Gelatine und den in dem vorherigen Schritt gebildeten Carbonylgruppen;
- Reduktion der im vorherigen Schritt gebildeten Imine zu Aminen.

14. Verfahren zur Produktion eines Produkts gemäß einem der Ansprüche 1-12 basierend auf hydrophiler Baumwolle, umfassend die folgenden Schritte:
- Bleichen und Hydrophilisieren der Baumwolle in einer der vermarkteten Formen: Faser, Garn, Gaze oder nicht gewebtes Gewebe;
- Oxidation der zuvor gebleichten und hydrophilisierten Baumwolle mit Umwandlung der in der Polymerkette der Baumwolle vorhandenen Hydroxylgruppen in Carbonylgruppen (Aldehyd oder Keton);
- Bildung von Iminen zwischen den im vorherigen Schritt gebildeten Carbonylgruppen und den Aminogruppen des Antibiotikums und der Gelatine und den in dem vorherigen Schritt gebildeten Carbonylgruppen;
- Reduktion der im vorherigen Schritt gebildeten Imine zu Aminen.

15. Verfahren nach einem der Ansprüche 13 oder 14, wobei das Bleichen der Baumwolle über die Reaktion der Baumwolle mit Wasserstoffperoxid, Natriumhydroxid und einem Detergens durchgeführt wird.

16. Verfahren nach einem der Ansprüche 13 oder 14, wobei die Oxidation der Baumwolle mit Perjodsäure oder einem Perjodat mit einer Konzentration, umfassend zwischen 3 und 10 g/l, bei einer Temperatur, umfassend zwischen 40 und 60 °C, für einen Zeitraum zwischen etwa 15 Minuten und 1 Stunde, und mit einem Reaktor-Flüssigphasenförderdruck zwischen 3 und 5 bar durchgeführt wird.

17. Verfahren nach einem der Ansprüche 13 oder 14, wobei die Bildung der Imine bei einer Temperatur, umfassend zwischen 40 und 60 °C, stattfindet mit Druckwerten, umfassend zwischen etwa 3 und 4 bar, und einem pH-Wert, umfassend zwischen 8,0 und 9,0, und mit Reaktionszeiten, umfassend zwischen 1 und 5 Stunden, und wobei das Antibiotikum in freier Form oder als ein Salz davon verwendet wird.

18. Verfahren nach Anspruch 14, wobei die Bildung der Imine durch Reaktion der Carbonylgruppen der Baumwolle und den Aminogruppen des Antibiotikums und der Gelatineproteine durchgeführt wird bei einer Temperatur, umfassend zwischen etwa 40 und 60 °C, mit Druckwerten, umfassend zwischen etwa 3 und 4 bar, bei einem pH-Wert, umfassend zwischen etwa 8,0 und 9,0, und mit Reaktionszeiten, umfassend zwischen 1 und 5 Stunden, und wobei das Antibiotikum in freier Form oder als ein Salz davon verwendet wird.

19. Verfahren nach Anspruch 18, wobei die Bildung der Imine durchgeführt wird unter Verwendung des Antibiotikums oder eines Salzes davon, welches zuvor in einer wässrigen Lösung von einer oder mehreren Gelatine(n) gelöst oder suspendiert wurde, wobei die Gesamtkonzentration genannter Gelatinen zwischen 0,5 und 3 Gew.-Vol-% umfasst.

20. Verfahren nach einem der Ansprüche 13 oder 14, wobei die Reduktion der Imine zu Aminen durchgeführt wird, indem zu der aus dem vorherigen Schritt stammenden Reaktionsmischung ein Reduktionsmittel, wie beispielsweise Borohydrid (NABH₄) oder Lithiumaluminiumhydrid (LiAlH₄) in der Form einer wässrigen Lösung mit einer Konzentration von 1 g/l hinzugegeben wird.

## Revendications

1. Produit basé sur du coton hydrophile, fonctionnalisé avec au moins un antibiotique d'aminoglycoside et au moins une gélatine, **caractérisé en ce que** l'antibiotique et la gélatine sont liés au coton par une liaison covalente, l'antibiotique est présent en une quantité comprise entre environ 4 et 45 mg par gramme de coton, et ledit produit est obtenu en faisant réagir le coton avec des quantités d'au moins une gélatine comprise entre 0,2 et 6 mg par gramme de coton.

2. Produit selon la revendication 1, où l'antibiotique est un ou plusieurs sélectionnés parmi la gentamicine, kanamycine, tobramycine, amikacine et néomycine.

3. Produit selon la revendication 1, où le coton est présent sous la forme d'une étoffe de fibres, de fils, de gaze ou d'étoffe non tissée.

4. Produit selon la revendication 3, où la quantité d'antibiotique est comprise entre 4 et 15 mg par gramme de coton lorsque ce dernier se présente sous la forme de fibres.

5. Produit selon la revendication 4, où ladite quantité d'antibiotique est comprise entre 6 et 10 mg par gramme de coton.

6. Produit selon la revendication 3, où la quantité d'antibiotique est comprise entre 14 et 45 mg par gramme de coton lorsque ce dernier se présente sous la forme d'une gaze.

7. Produit selon la revendication 6, où ladite quantité d'antibiotique est comprise entre 18 et 30 mg par gramme de coton.

8. Produit selon la revendication 1, où lesdites gélatines sont obtenues de la peau bovine ou porcine ou de parties osseuses porcines.

9. Produit selon la revendication 1, où la quantité de gélatines est comprise entre 0,2 et 5 mg par gramme de coton lorsque ce dernier se présente sous la forme de fibres.

10. Produit selon la revendication 9, où ladite quantité de gélatines est comprise entre 0,5 et 1 mg par gramme de coton.

11. Produit selon la revendication 1, où la quantité de gélatines est comprise entre 0,3 et 6 mg par gramme de coton lorsque ce dernier se présente sous la forme de gaze.

12. Produit selon la revendication 11, où ladite quantité de gélatines est comprise entre 0,7 et 1,2 mg par gramme de coton.

13. Procédé de fabrication d'un produit selon l'une quelconque des revendications précédentes, basé sur du coton hydrophobe, comprenant les étapes suivantes:
- blanchiment du coton selon l'une des formes commercialisée: fibre, fil, gaze ou étoffe non tissée;
- oxydation du coton blanchi préalablement, avec la transformation des groupes hydroxyle présents dans la chaîne de polymères du coton en groupes carbonyle (aldéhyde ou cétone);
- formation d'imines entre les groupes carbonyle formés à l'étape précédente et les groupes aminés de l'antibiotique et de la gélatine;
- réduction des imines formées à l'étape précédente en amines.

14. Procédé de fabrication d'un produit selon l'une quelconque des revendications 1 à 12, basé sur du coton hydrophile, comprenant les étapes suivantes:
- blanchiment et hydrophilisation du coton en une des formes commercialisées: fibre, fil, gaze ou étoffe non tissée;
- oxydation du coton préalablement rendu hydrophile et blanchi, avec la transformation des groupes hydroxyle présents dans la chaîne de polymères du coton en groupes carbonyle (aldéhyde ou cétone);
- formation d'imines entre les groupes carbonyles formés à l'étape précédente et les groupes aminés de l'antibiotique et de la gélatine;
- réduction des imines formées à l'étape précédente en amine.

15. Procédé selon l'une quelconque des revendications 13 ou 14, où le blanchiment du coton est exécuté en faisant réagir le coton avec du peroxyde d'hydrogène, de la soude caustique et un détergent.

16. Procédé selon l'une quelconque des revendications 13 ou 14, où l'oxydation du coton est exécutée avec l'acide périodique ou un périodate avec une concentration comprise entre 3 et 10 g/l, à une température comprise entre 40 et 60°C, pendant des périodes comprises entre environ 15 minutes et 1 heure, et avec des pressions d'amenée en phase liquide du réacteur entre 3 et 5 bar.

17. Procédé selon l'une quelconque des revendications 13 ou 14, où la formation des imines a lieu à une température comprise entre environ 40 et 60°C, les valeurs de pression étant comprises entre environ 3 et 4 bar, à un pH compris entre 8,0 et 9,0 et avec des temps de réaction compris entre 1 et 5 heures, et où l'antibiotique est utilisé sous forme libre ou sous forme de sel de celui-ci.

18. Procédé selon la revendication 14, où la formation des imines est exécutée en faisant réagir les groupes carbonyle du coton et les groupes aminés de l'antibiotique et des protéines de gélatine à une température comprise entre environ 40 et 60°C, les valeurs de pression étant comprises entre environ 3 et 4 bar, à un pH compris entre 8,0 et 9,0, et avec des durées de réaction comprises entre 1 et 5 heures, et où l'antibiotique est utilisé sous forme libre ou sous forme de sels de celui-ci.

19. Procédé selon la revendication 18, où la formation des imines est exécutée en utilisant l'antibiotique ou un sel de celui-ci préalablement dissous ou suspendu dans une solution aqueuse d'une ou de plusieurs gélatines, où la concentration totale desdites gélatines est comprise entre 0,5 et 3% w/v.

20. Procédé selon l'une quelconque des revendications 13 ou 14, où la réduction des imines en amines est exécutée en ajoutant au mélange de réaction résultant de l'étape précédente un agent réducteur comme un borohydrure de sodium (NaBH₄) ou un hydrure de lithium aluminium (LiAlH₄) sous la forme de solutions aqueuses ayant une concentration de 1 g/l.
